# EUROPÄISCHE PATENTSCHRIFT

(11) **EP 0 681 912 B1**
(45) Veröffentlichungstag und Bekanntmachung des Hinweises auf die Patenterteilung: **13.06.2001**
(21) Anmeldenummer: 95106756.0
(22) Anmeldetag: 04.05.1995
(51) Int. Cl.: B32B 25/12, B05D 1/18, C09D 109/10, A61F 6/04, A41D 19/00, A61M 25/00

(54) **Flexibler Gummiartikel und Verfahren zu dessen Herstellung**
Flexible rubber product and manufacturing process therefor
Produit de caoutchouc flexible et procédé de fabrication

(30) Priorität: 13.05.1994 AT 99194
(43) Veröffentlichungstag der Anmeldung: 15.11.1995
(73) Patentinhaber: Semperit Aktiengesellschaft Holding, 1031 Wien (AT)
(72) Erfinder: Wukovnig, Siegfried, Dipl.-Ing. Dr., A-2624 Breitenau a. Steinfelde (AT); Holzner, Armin, Dipl.-Ing. Dr., A-2830 Ternitz (AT)
(74) Vertreter: Müller, Hans-Jürgen, Dipl.-Ing.

(56) Entgegenhaltungen:
- EP-A- 0 113 526
- EP-A- 0 574 160
- EP-A- 0 594 410
- EP-B- 0 105 613
- WO-A-84/00908
- DE-C- 2 828 617
- US-A- 3 411 982
- US-A- 3 856 561
- US-A- 3 967 014
- US-A- 4 100 309
- US-A- 4 482 577

## Beschreibung

Die Erfindung betrifft einen flexiblen Gummiartikel, mit einem de Schlüpfrig- keit gegenüber menschlicher Haut sowohl im nassen als auch im trockenen Zustand verbesseruden polymeren Überung jowei ein Herstellungs verfahren für dieser und bevorzugte Verwendungen.

Die meisten an der Oberfläche unbehandelten Gummiartikel weisen einen relativ großen Reibungswiderstand gegenüber menschlicher Haut auf. So sind zum Beispiel unbehandelte Gummihandschuhe kaum anzuziehen. Um das Anziehen dieser Handschuhe zu erieichtern, wird konventionellerweise Puder (z.B. Stärkepuder, usw.) als Gleitmittel auf der Innenschicht des Handschuhes aufgebracht. Die Verwendung von Puder ist jedoch insbesonders im medizinischen Bereich problematisch, da Puderreste, falls sie in offene Wunden gelangen, zur Granulombildung führen können. Ein weiterer wichtiger Anwendungsbereich für puderfreie Handschuhe liegt in der Elektronikindustrie.

Die konventionelle Methode, Gummiprodukte und im speziellen Handschuhe gegenüber der menschlichen Haut ohne Verwendung von Puder schlüpfrig zu machen, besteht in der Oberflächenchlorierung. Hierbei wird das Gummiprodukt mit wäßrigen, chlorgashaltigen Medien behandelt und anschließend gewaschen. Dabei wird vor allem eine gute Gleitfähigkeit gegenüber der trockenen menschlichen Haut erreicht, jedoch wird durch diese Oberflächenchlorierung die Polymerstruktur künstlich gealtert und es kommt so zu einer deutlichen Verschlechterung der physikalischen und chemischen Eigenschaften des Produktes (Festigkeit, Reißdehnung, Wasserquellung, Lagerfähigkeit, etc.). Durch eine derartige Oberflächenbehandlung wird jedoch kaum eine verbesserte Naßanziehbarkeit der Handschuhe erreicht.

Eine alternative Oberflächenbehandlung stellt die Beschichtung mit Hydrogelen dar. Einige derartige Hydrogele sind schon seit langem bekannt. Dies sind z.B. Polyurethane, Polyvinylpyrrolidon, Polyhydroxyethylacrylat oder -methacrylat, Polyhydroxypropylacrylat oder -methacrylate und Copolymere dieser (Meth)-acrylaten miteinander oder mit Acryl-oder Methacrylsäure, Acryl- oder Methacrylester oder Vinylpyridin.

Aus der US 3 813 695 A ist ein getauchter Gummihandschuh bekannt, der an der Innenseite mit einem Hydrogelpolymer, wie zum Beispiel Polyvinylpyrrolidon, Polyhydroxyethyl-acrylat oder -methacrylat, Polyhydroxypropyl-acrylat oder -methacrylat und Copolymere dieser (Meth)Acrylaten untereinander oder mit Acryl- oder Methacrylsäure. Das bevorzugte Hydrogelpolymer ist ein Copolymer von 2-Hydroxyethylmethacrylat mit Methacrylsäure oder mit 2-Ethylhexylacrylat oder ein ternäres Copolymer von 2-Hydroxyethylmethacrylat, Methacrylsäure und 2-Ethylhexylacrylat.

Die US 4 482 577 A legt einen Prozeß für die Beschichtung eines flexiblen, vulkanisierten Operationshandschuhs mit einem hydrophilen Polymer offen, wodurch die Aufbringung von Puder an der Handschuhinnenseite unterlassen werden kann. Das hierbei verwendete Copolymer besteht aus einer Mischung von 2-Hydroxyethylmethacrylat- und 2-Ethylhexylacrylat-Einheiten.

Die US 4 100 309 A betrifft die Aufbringung einer gleitfähigen Schicht aus einem Polyurethan-Polyvinylpyrrolidon-Komplex.

Üblicherweise wird bei diesen Produkten bzw. Verfahren das Hydropolymer gemeinsam mit einem Härtungsmittel in gelöster Form auf das Produkt aufgebracht, anschließend das Lösungsmittel durch Trocknung entfernt, wobei die Polymerschicht ausgehärtet und so eine Hydrogelschicht ausgebildet wird. Hierbei werden oft organische Lösungsmittel und gesundheitlich nicht unbedenkliche monomere oder oligomere Vemetzungsmittel verwendet.

Weitere bereits bekannte Lösungen beschreiben den Einsatz von Vinylidenhalogenid-bzw. Vinylhalogenidlatices zur Herstellung einer Gleitschicht auf Gummiartikel (z.B. US. 5 069 965 A), wo die Gleitfähigkeit meist chlorhaltiger Polymere ausgenutzt wird. Sehr oft ist jedoch die Präsenz von chlorhaltigen Polymeren insbesondere bei Artikeln zur einmaligen Anwendung unerwünscht.

Weitere Druckschriften betreffen den Einsatz von diversen Latexgemischen für eine Gleitschichtherstellung (z.B. DE 26 28 059 C2 und US 4 082 826 A). Hier kommen insbesondere Gemische aus mindestens zwei Latextypen zum Einsatz, wobei ein Latextyp mit einer hohen Dehnung als Haftvermittler und ein Latextyp mit hoher Härte bzw. geringer Dehnung zum Erhalt einer ausreichenden Gleitfähigkeit dient. Diese Rezepturen sind üblicherweise ein Kompromiß zwischen Gleitfähigkeit und ausreichender Filmflexibilität und Haftung des Films am Gummiprodukt. Des weiteren ergeben sich hier oft Probleme mit in der Praxis komplizierten Rezepturen und hohen Preisen der verwendeten Latices.

Der Gegenstand der Erfindung ist in den Ansprüchen 1 und 5 definiert.

Gemäß der vorliegenden Erfindung besteht der polymere Überzug aus einem einzigen Copolymerlatextyp, welcher auf den gummiartikel aufkoaguliert wird und einen wesentlichen Anteil an hydrophilen Monomerbausteinen enthält.

Dieser hydrophile Anteil besteht vorzugsweise aus Acrylsäure bzw. Methacrylsäure oder aus Acrylaten bzw. Methacrylaten, beziehungsweise einem Gemisch dieser Monomerbausteine, jedoch können auch andere hydrophile bzw. polare Polymerbausteine verwendet werden.

Im Unterschied zum heutigen Stand der Technik wird in der vorliegenden Erfindung diese Gleitschicht nicht durch Auftragen einer mit einem Härter versetzten Polymer- oder Monomerlösung hergestellt, sondern das Verfahren zeichnet sich dadurch aus, daß die polymere Gleitschicht in Form eines Copolymerlatex, der wesentliche Anteile an hydrophilen Bestandteilen enthält, aufgebracht wird. Der verwendete Latex kann sowohl unvernetzt als auch im vorvemetzten Zustand vorliegen. So entfällt die Härtung bzw. Vernetzung nach dem Tauchprozeß. Die aus dem erfindungsgemäßen Copolymerlatex bestehende Gleitschicht wird entsprechend der vorliegenden Erfindung durch Koagulation des erfindungsgemäßen Latex mittels mehrwertiger Metallionen, mittels wärmesensibler Koagulantien oder mittels Wärme und/oder Trocknung auf der Form hergestellt. Diese Koagulantien können entweder in einem unmittelbar vorangehenden Tauchschritt aufgetragen werden oder im Falle der Koagulation durch mehrwertige Metallionen auch durch Diffusion durch die erste Trägerschicht an der Oberfläche dieser Trägerschicht verfügbar gemacht werden. Diese Art der Aufbringung einer Gleitschicht unterscheidet sich von den bisher bekannten Verfahren, die die polymere Gleitschicht aus einer Lösung aufbringen und anschließend mit einem System aus Harzen mit der ersten Schicht oder in sich selbst vernetzen. Dabei verwendete Harzsysteme sind zum Beispiel Melamin-Formaldehydharze, deren Anteil an monomeren Formaldehyd eine Anwendung im medizinischen Bereich nicht erstrebenswert erscheinen läßt.

Im Unterschied zu bekannten Verfahren, wo die Gleitschicht in Latexform aufgebracht wird, kann auf chlorhältige Polymere verzichtet werden, bzw. ist es möglich mit einem Latextyp, welcher durch Koagulation an die Oberfläche des Trägermaterials gebunden wird, das Auslangen zu finden.

Gemäß einer vorteilhaften Weiterentwicklung der vorliegenden Erfindung können mit der Gleitschichte gleichzeitig ein oberflächenaktiver Stoff und/oder Siloxane angewendet werden. Dadurch kann eine verbesserte Naßanziehbarkeit des Handschuhes erreicht werden. Als oberflächenaktive Stoffe können sowohl anionische, kationische als auch nichtionogene Tenside verwendet werden. Bevorzugt können kationische Tenside oder Siloxane verwendet werden, die im Unterschied zu bereits bekannten Vorschlägen in der Gleitschichtdispersion eingemischt sind und gleichzeitig mit der polymeren Gleitschicht aufgebracht werden. Die Substanzen befinden sich hier verteilt innerhalb der gesamten Gleitschicht und es kann in der Gleitschichtherstellung auf einen zusätzlichen Verfahrensschritt verzichtet werden.

Gegenstand der vorliegenden Erfindung ist, wie oben erwähnt, ein flexibler Gummiartikel, an dessen Oberfläche ganz oder teilweise eine Gleitschicht angebracht wurde, welche Schlüpfrigkeit gegenüber der menschlichen Haut sowohl im nassen als auch im trockenen Zustand gewährleistet. Gummiartikel, bei welchen eine gute Gleitfähigkeit mit der menschlichen Haut erforderlich sind, existieren in großer Vielfalt und beinhalten beispielsweise Handschuhe, im speziellen medizinische Operations- und Untersuchungshandschuhe, Katheder, Kondome, Fingerlinge und Badehauben.

Vorzugsweise handelt es sich dabei um Artikel, die durch Tauchen einer Form in eine Latexmischung erhalten werden, wobei die Latextauchmischung sowohl auf Naturlatex als auch auf Syntheselatex basieren kann. Die verwendeten Latextauchmischungen können sowohl unvernetzt als auch im vorvemetzten Zustand vorliegen. Im Falle einer unvernetzten oder nur partiell vorvernetzten Latextauchmischung muß die erhaltene Gummischicht noch an der Tauchform vulkanisiert werden.

Vorzugsweise werden Latextauchmischungen verwendet, die vemetzte oder partiell vorvemetzte Polymere enthalten. Dieser Latex wird auf die Tauchform, welche die Gestalt des Endproduktes vorgibt, aufgebracht und koaguliert und erzeugt so eine dünne Schicht aus elastischem, relativ resistentem Gummimaterial. Die Latextauchmischung enthält die üblichen Compoundierungszutaten, wie beispielsweise Schwefel, Zinkoxid, organische Beschleuniger (u.a. Zinksalze von Dithiocarbamaten, Thiurame, Thioharnstoff), Stabilisatoren, Wachse, Alterungsschutzmittel, Viskositätsregler, Füllstoffe und Farben. Als Latex für die Trägerschicht kann sowohl Naturkautschuk, als auch Synthesekautschuk, der für die Anwendung in Tauchverfahren geeignet ist, verwendet werden. Von den natürlichen und synthetischen Latices werden vorzugsweise Naturkautschuk, Polychloropren, synthetisches Polyisopren, Nitrilbutadien- und Styrolbutadienkautschuk bzw. eine Mischung dieser Polymere verwendet. Rezepturen zur Verarbeitung solcher Latices sind bekannt und Personen, die in der Kunst der Herstellung derartiger Artikel bewandert sind, können Rezepturen, Bedingungen für die Vernetzung derartiger Latices und Tauchprozeßparameter den speziellen Anforderungen des Endproduktes anpassen.

Nach der vorliegenden Erfindung werden die erfindungsgemäßen flexiblen Gummiartikel an ihren Oberflächen oder Teilen ihrer Oberflächen mit einer Gleitschicht, welche keine staub- oder puderförmigen Teilchen enthält, versehen. Diese Gleitschicht ist notwendig, um Schlüpfrigkeit gegenüber der menschlichen Haut zu erreichen. Dadurch wird die Anziehbarkeit vieler Produkte gewährleistet (z. B. eng anliegende Handschuhe). Des weiteren stellt eine gute Schlüpfrigkeit gegenüber menschlichem Gewebe ein wesentliches Funktionsmerkmal einiger spezieller Artikel dar (z.B. Katheder).

Im speziellen Fall der Herstellung von Tauchartikel wird die Oberfläche der Produkte üblicherweise in zwei Bereiche unterteilt: Die Tauchformseite, welche in ihrer Rauheit der Morphologie der Form entspricht und die Tauchbadseite, welche sich während des Tauchvorganges außen befindet. Diese ist üblicherweise glatt und gelangt beim Abziehen des dünnwandigen Gummiproduktes von der Form üblicherweise an die Innenseite des Produktes (z.B. Handschuhinnenseite). Oft müssen beide Seiten des Produktes beim Herstellungsprozeß mit Gleit- bzw. Trennmittel behandelt werden, um ein Zusammenkleben des Produktes zu vermeiden und andererseits (z.B. im Fall von Handschuhen), ein reibungsloses Anziehen zu gewährleisten. Gemäß der vorliegenden Erfindung kann die Gleitschicht wahlweise tauchformseitig, tauchbadseitig oder auf beiden Seiten der Tauchprodukte aufgetragen werden.

Die erfindungsgemäße Gleitschicht besteht aus einem Copolymeren, welches stark hydrophile Polymeranteile enthält. Diese bestehen vorzugsweise aus Acrylsäure bzw. Methacrylsäure, aus Acrylaten bzw. Methacrylaten, beziehungsweise aus einem Gemisch dieser Monomerbausteine des Polymers, wobei auch andere stark hydrophile bzw. polare Polymerbausteine, wie z.B. Vinylpyrrolidon, Vinylalkohol gut geeignet sind. Zusätzlich können auch hydrophobe bzw. weniger wasseraufnehmende Anteile im Copolymer vorhanden sein, vorzugsweise Styrol, Butadien, Isopren und/oder Acrylnitril. Das erfindungsgemäß zu verwendende Copolymer liegt zur Verarbeitung in Form eines Copolymerlatex vor und wird nach dem Auftragen auf das Produkt oder auf die Tauchform koaguliert. Der erfindungsgemäß einzusetzende Copolymerlatex wird vorzugsweise durch Sprühen, Tauchen, Streichen oder andere geeignete Verfahren auf die Oberfläche des Gummiartikels oder der Tauchform gebracht.

Um eine in manchen Fällen auftretende Ablösung dieser Gleitschicht von der Oberfläche des erfindungsgemäßen Gummiartikels zu vermeiden, wird diese an der Oberfläche des Artikels mittels eines geeigneten Koagulationsverfahren gebunden. Die Koagulation kann sowohl durch Wärme und/oder Trocknung, durch Zugabe von wärmesensiblen Koagulantien oder auch durch andere Koagulantien (z.B. Salze mehrwertiger Kationen, Säuren, u.a.) herbeigeführt werden. Die angeführten chemischen Koagulantien können entweder direkt dem erfindungsgemäß einzusetzende Copolymerlatex vor dem Auftragen zugegeben werden oder das Produkt bzw. die Tauchform kann unmittelbar vor dem Aufbringen der Gleitschicht mit dem Koagulant vorbehandelt werden oder es können Reste der für die Koagulation des Gummifilms (Trägerschicht) verwendeten Substanzen an die Oberfläche der Trägerschicht diffundieren und mit dem für die Gleitschicht verwendeten Latex reagieren. Umgekehrt ist eine Bindung einer formseitig aufgebrachten Gleitschicht durch Reaktion der produktbildenden Latexschicht mit überschüssigem Koagulant, welches aus der Gleitschicht herrührt, möglich.

Eine weitere Ausführung der vorliegenden Erfindung betrifft die Anwendung von oberflächenaktiven Substanzen bzw. Siloxanen in der erfindungsgemäßen Gleitschicht. Abhängig vom Polymertyp des Produktes und der Gleitschicht, der Dicke und dem verwendeten Koagulant in der Gleitschicht und den erforderlichen Gleiteigenschaften des Produktes ist es, insbesondere zur Erhöhung der Naßgleitfähigkeit, manchmal vorteilhaft, oberflächenaktive Substanzen in die erfindungsgemäße Gleitschicht einzubringen.

Hierbei können sowohl anionische, kationische als auch nichtionogene Tenside aber auch Siloxane verwendet werden. Besonders gute Verbesserungen der Gleitfähigkeit werden hierbei oft durch zusätzliches Aufbringen von Tensiden aus der Gruppe der höher substituierten Amine oder von Siloxanen erreicht. Hier können z.B. quartäre Amintypen zur Anwendung gelangen, welche nicht nur die Gleitfähigkeit der Handschuhe verbessern, sondern auch gut hautverträglich sind und bakterizide und fungizide Eigenschaften aufweisen. So ergibt sich für den Anwender im medizinischen Bereich nicht nur ein Produkt mit idealen Gleiteigenschaften, sondern auch mit einem verbesserten Infektionsschutz. Diese erfindungsgemäß einzusetzenden Tenside werden gleichzeitig mit der Gleitschicht auf den Gummiartikel aufgetragen.

Nachfolgend wird nun die Erfindung anhand einer ausführlichen Beschreibung des Copolymeren der Gleitschicht und anhand von Beispielen näher erläutert:

Die Gleitschicht besteht aus einem Copolymeren, welcher einen hydrophilen Anteil enthält. Unter dem Begriff Copolymeren wird dabei nicht nur ein Gemisch aus zwei Monomerbausteinen, sondern auch ein Gemisch aus bis zu fünf Monomerbausteinen verstanden. Der erwähnte hydrophile Anteil enthält vorzugsweise Methacryl- bzw. Acrylsäure und/oder deren Ester mit ein- oder mehrwertigen Alkoholen.

Der Rest R der Alkoholfunktion (allg. Formulierung ROH) dieser Ester umfaßt beispielsweise Alkylgruppen (Methyl, Propyl, Butyl), substituierte Alkylgruppen (2-Methylpentyl, 2-Ethylpentyl, 2-Propylpentyl, 2-Ethylhexyl, 2-Propylhexyl), Hydroxyalkylgruppen (z.B. 2-Hydroxyethyl, 2-Hydroxypropyl, 3-Hydroxypropyl, 4-Hydroxybutyl), verzweigte bzw. unverzweigte Hydroxyoligo- bzw. Hydroxypolyether.

Der hydrophile bzw. polare Anteil kann auch aus Polyamiden, Polyestern, Polyaminen, Polyurethane oder Polyether bestehen. Diese Polymere können unter Umständen selbstvernetzende Gruppen enthalten.

Der hydrophile Anteil des Copolymeren zeichnet sich dadurch aus, daß auf chlor- bzw. halogenhaltige Bestandteile verzichtet werden kann.

Es kann auch ein hydrophober bzw. weniger wasseraufnehmender Polymeranteil vorhanden sein. Dieser besteht vorzugsweise aus Polystyrol, Polybutadien, Polyisopren oder Polyacrylnitril bzw. Copolymeren entsprechender Monomerbausteine.

Beispiele für erfindungsgemäß ausgeführte flexible Gummiartikel:

### Beispiel 1:

Die zur Herstellung von Latexhandschuhen verwendeten Tauchformen werden vor dem Tauchprozeß gereinigt und getrocknet. Danach wird in eine Koagulationslösung, welche Kalziumnitrat, Polyethylenglykol (MG ca. 6000) und einen wasserlöslichen Silikontyp enthält, getaucht und kurz mit Heißluft getrocknet. Anschließend wird zum Aufbringen des Handschuhs in die vorvernetzte Naturlatexmischung getaucht. Nach neuerlichem kurzen Trocknen wird in ein Tauchbad, welches einen Acrylat-Copolymerlatex (z.B. Hycar 26083, Fa. BFGoodrich) mit 20% Trockengehalt zum Auftragen der Gleitschicht getaucht, wobei es zu einer Bindung der Gleitschicht an den Naturlatexfilm durch Koagulation des Polymeriatex mittels Kalziumionen, die an die Oberfläche der Trägerschicht diffundiert sind, kommt. Nach neuerlichem kurzen Trocknen wird mit Heißwasser gewaschen, danach 20 Minuten bei 120° C fertiggetrocknet und vulkanisiert. Am Ende des Herstellprozesses wird der Handschuh nach mit Silikonemulsion behandelt, von der Form abgezogen (wobei die aufgetragene Gleitschicht an die Handschuhinnenseite gelangt) und nachgetrocknet. Das Produkt weist sehr gute Anziehbarkeit mit trockenen Händen und eine ausreichend gute Anziehbarkeit mit nassen Händen auf.

### Beispiel 2:

Es wird Beispiel 1 wiederholt, mit dem Unterschied, daß durch die Wahl anderer Tauchformen Gummifingerlinge hergestellt werden.

### Beispiel 3:

Es wird Beispiel 1 wiederholt, mit dem Unterschied, daß vorvernetzter Chloroprenlatex (Neoprene Latex 750, Firma Du Pont) für die Herstellung der Trägerschicht verwendet wird.

### Beispiel 4:

Es wird Beispiel 1 wiederholt mit dem Unterschied, daß am Ende des Herstellungsprocesses statt der Silikonemulsion ein Polyglykol-Polyamin-Kondensationsharzes (Polyquart H81, Firma Henkel) aufgetragen wird mit dem Ergebnis, daß sich im Vergleich zum Beispiel 1 die Naßanziehbarkeit des Handschuhs verbessert.

### Beispiel 5:

Es wird Beispiel 1 wiederholt mit dem Unterschied, daß am Ende des Herstellungsprocesses statt der Silikonemulsionsbehandlung des Handschuks in ein, mit 2% Lauryltrimethylammoniumchlorid (Dehyquart LT, Firma Henkel) getaucht wird. Danach folgt kurze Trocknung der Handschuchs an der Form und Abzielen des Fertigprodukts. Neben guter Anziehbarkeit des Handschuhes mit nassen und trockenen Händen wird durch die bakteriziden Eigenschaften des quartären Amins ein zusätzlicher Schutz gegen Infektionen für den Anwender erreicht.

### Beispiel 6:

Es wird Beispiel 1 wiederholt, mit dem Unterschied, daß unter Erhalt vergleichbarer Eigenschaften des Endproduktes statt dem Acrylat-Copolymerlatex ein Acrylsäureester-Acrylnitril-Copolymerlatex, der selbstvernetzende Gruppen enthält (Acralen AFR, Fa. Bayer), eingesetzt wird.

### Beispiel 7:

Dünnwandige Latexhandschuhe werden durch Tauchen von Porzellanformen in eine Naturlatexmischung hergestellt. Nach dem Aufbringen der Latexschicht werden die Handschuhe bei 85° C mit Wasser ausgewaschen. Anschließend wird in ein Tauchbad mit folgender Zusammensetzung getaucht:
- Rhoplex AC-33 (Acrylathaltiger Latex, Trockengehalt 46%, Fa. Rohm & Haas): 20,7 Teile,
- Coagulant AW (Organopolysiloxan, Firma Bayer): 0,1 Teile,
- Marlamid AS18 (Stearinsäure-Alkanolamin-Kondensationsprodukt, Firma Hüls): 5 Teile,
- Essigsäure 98%: 0,2 Teile,
- Wasser: 74 Teile.

Danach wird der Handschuh 25 Minuten in einem Heißluftofen bei 120° C getrocknet und vulkanisiert. Danach wird der fertige Handschuh von der Form abgezogen.
Das Produkt weist gute Anziehbarkeit, sowohl mit nassen als auch mit trockenen Händen, auf.

### Beispiel 8:

Es wird Beispiel 7 wiederholt, mit dem Unterschied, daß durch Auswahl unterschiedlicher Tauchformen, Vortauchbad- und Naturlatextauchbadrezepturen Kondome hergestellt werden.

### Beispiel 9:

Es wird Beispiel 7 wiederholt, mit dem Unterschied, daß zur Herstellung des Handschuhes im Tauchverfahren Chloroprenlatexmischung (Neoprene Latex 750, Firma Du Pont) verwendet wird.

### Beispiel 10:

Dünnwandige Latexhandschuhe werden durch Tauchen von Porzellanformen in eine Naturlatexmischung hergestellt. Nach dem Aufbringen der Latexschicht werden die Handschuhe bei 85° C mit Wasser ausgewaschen. Anschließend wird in ein Tauchbad mit verdünnter Salzsäure, danach in ein Bad mit Acralen ATR-Latex (Styrol-Acrylsäure-Copolymeriatex, Fa. Bayer, Trockengehalt 48%) mit 15% Trockensubstanz getaucht und nochmals mit Heißwasser gewaschen. Danach wird der Handschuh 25 Minuten in einem Heißluftofen bei 120° C getrocknet und vulkanisiert. Schließlich wird der fertige Handschuh von der Form abgezogen, mit Silikonemulsion (Polydimethylsiloxan) behandelt und nachgetrocknet, Das Produkt weist gute Anziehbarkeit, insbesondere mit trockenen Händen, auf.

### Beispiel 11:

Es wird ein Naturlatexhandschuh hergestellt, welcher auf beiden Seiten mit der erfindungsgemäßen Gleitschicht versehen ist. Dazu werden die Formen nach vorhergehender Reinigung und Vorwärmen in ein Tauchbad mit folgender Rezeptur getaucht:
- Acralen ATR (Trockengehalt 48%): 41,2 Teile,
- Coagulant AW (Organopolysiloxan, Firma Bayer): 0,2 Teile,
- Marlamid AS18 (Stearinsäure-Alkanolamin-Kondensationsprodukt, Firma Hüls): 2,5 Teile,
- Essigsäure 98%: 0,1 Teile,
- Wasser: 56 Teile.

Anschließend wird mit Heißluft zwischengetrocknet und in eine partiell vorvemetzte Naturlatextauchmischung mit einem Feststoffgehalt von 50%, welche ebenfalls mittels einem Organopolysiloxan wärmesensibilisiert wurde, getaucht. Dadurch wird eine Naturlatexträgerschicht gebildet, welche analog zu Beispiel 7 sodann an der Tauchbadseite mit einer erfindungsgemäßen Gleitschicht versehen wird. Die Vulkanisation erfolgt in einem Heißluftofen über 30 Minuten bei 120° C. Anschließend wird der Handschuh abgekühlt und von der Form abgezogen. Das Produkt weist die erfindungsgemäße Gleitschicht mit den bereits beschriebenen Eigenschaften an beiden Seiten des Handschuhs auf.

## Patentansprüche

1. Flexibler Gummiartikel mit einem die Schlüpfrigkeit verbessernden polymeren Überzug an zumindest einem Oberflächenteil des Gummiartikels,
**dadurch gekennzeichnet**,
daß der polymere Überzug einen Copolymerlatex aufweist, welcher aus einem einzigen Copolymer besteht, der neben stark hydrophilen bzw. polaren Anteilen auch wenig wasseraufnehmende oder hydrophobe Anteile enthält, wobei die hydrophilen bzw. polaren Anteile aus Acrylbzw. Methacrylsäure und/oder deren Ester mit ein- bzw. mehrwertigen Alkoholen, Acrylaten bzw. Methacrylaten, Vinylpyrrolidon, Vinylalkohol, Vinylacetat, Vinylester, Polyamine, Polyurethane und/oder Polyether bestehen, und wobei die weniger wasseraufnehmenden bzw. hydrophoben Anteile Styrol, Butadien, Isopren und/oder Acrylnitril aufweisen, und
daß der polymere Überzug durch Koagulation des Copolymerlatex an der Oberfläche des Gummiartikels gebunden ist.

2. Gummiartikel nach Anspruch 1,
**dadurch gekennzeichnet**,
daß der polymere Überzug eine Substanz mit oberflächenaktiven, bakteriziden, fungiziden und/oder keimwachstums- hemmenden Eigenschaften aufweist und/oder mit einer solchen überzogen ist.

3. Gummiartikel nach Anspruch 2,
**dadurch gekennzeichnet**,
daß die oberflächenaktive Substanz Silikone, langkettige Fettsäureamine, sekundäre und/oder tertiäre Amine, quartäre Amine mit bakteriziden und/oder keimwachstumshemmenden Eigenschaften, Siloxane, Salze von organischen Säuren und/oder Kondensationsprodukte von organischen Säuren oder Alkoholen mit mehrwertigen Alkoholen aufweist.

4. Gummiartikel nach einem der Ansprüche 2 oder 3,
**dadurch gekennzeichnet**,
daß quartäre Amintypen als bakterizide, fungizide und/oder keimwachstumshemmende Substanz zusätzlich aufgebracht sind.

5. Verfahren zur Herstellung eines flexiblen Gummiartikels nach einem der Ansprüche 1-4,
**dadurch gekennzeichnet**,
daß ein den Überzug bildendes Mittel durch Tauchen oder Sprühen auf den Gummiartikel oder einen Vorläufer desselben aufgebracht und anschließend zum Koagulieren veranlaßt wird.

6. Verfahren nach Anspruch 5,
**dadurch gekennzeichnet**,
daß das Koagulieren durch Erwärmen bzw. Trocknen vorgenommen wird.

7. Verfahren nach Anspruch 6,
**dadurch gekennzeichnet,**
daß Organosiloxane als wärmesensible Verbindung zum Wärmekoagulieren verwendet werden.

8. Verfahren nach einem der Ansprüche 5 - 7,
**dadurch gekennzeichnet,** daß dem Mittel chemische Koagulantien zugesetzt werden.

9. Verfahren nach Anspruch 8,
**dadurch gekennzeichnet,**
daß Salze mehrwertiger Kationen als chemische Koagulantien verwendet werden.

10. Gummiartikel nach einem der Ansprüche 1-4,
**dadurch gekennzeichnet,** daß der Gummiartikel ein Handschuh ist.

11. Gummiartikel nach einem der Ansprüche 1-4,
**dadurch gekennzeichnet,** daß der Gummiartikel ein Kondom ist.

12. Gummiartikel nach einem der Ansprüche 1-4,
**dadurch gekennzeichnet,** daß der Gummiartikel ein Katheter ist.

## Claims

1. Flexible rubber article with a polymer coating improving slipperiness on an at least one part of the surface of the rubber article, characterised in that the polymer coating comprises a copolymer latex which consists of a single copolymer which in addition to highly hydrophilic or polar components also contains minimally water-absorbent or hydrophobic components, wherein the hydrophilic or polar components consist of acrylic or methacrylic acid and/or their esters with monovalent or polyvalent alcohols, acrylates or methacrylates, vinyl pyrrolidone, vinyl alcohol, vinyl acetate, vinyl ester, polyamines, polyurethanes and/or polyether, and wherein the minimally water-absorbent or hydrophobic components comprise styrene, butadiene, isoprene and/or acrylonitrile, and in that the polymer coating is bound to the surface of the rubber article by coagulation of the copolymer latex.

2. Rubber article according to claim 1, characterised in that the polymer coating comprises and/or is coated with a substance with surface-active, bactericidal, fungicidal and/or germ growth-inhibiting properties.

3. Rubber article according to claim 2, characterised in that the surface-active substance comprises silicones, long-chain fatty acid amines, secondary and/or tertiary amines, quaternary amines with bactericidal and/or germ growth-inhibiting properties, siloxanes, salts of organic acids and/or condensation products of organic acids or alcohols with polyvalent alcohols.

4. Rubber article according to one of claims 2 or 3, characterised in that quaternary amine types are additionally applied as bactericidal, fungicidal and/or germ growth-inhibiting substance.

5. Method for production of a flexible rubber article according to one of claims 1 to 4, characterised in that an agent forming the coating is applied to the rubber article or a precursor thereof by dipping or spraying and then made to coagulate.

6. Method according to claim 5, characterised in that the coagulation is carried out by heating or drying.

7. Method according to claim 6, characterised in that organosiloxanes are used as heat-sensitive compound for thermal coagulation.

8. Method according to one of claims 5 to 7, characterised in that chemical coagulants are added to the agent.

9. Method according to claim 8, characterised in that salts of polyvalent cations are used as chemical coagulants.

10. Rubber article according to one of claims 1 to 4, characterised in that the rubber article is a glove.

11. Rubber article according to one of claims 1 to 4, characterised in that the rubber article is a condom.

12. Rubber article according to one of claims 1 to 4, characterised in that the rubber article is a catheter.

## Revendications

1. Article en caoutchouc flexible, muni d'un revêtement polymère améliorant l'onctuosité sur au moins une partie de la surface dudit article en caoutchouc, caractérisé en ce que le revêtement polymère comporte un latex copolymère constitué d'un unique copolymère qui, outre des fractions fortement hydrophiles et/ou polaires, contient également de petites fractions peu hygroscopiques ou hydrophobes, les fractions hydrophiles ou polaires étant constituées d'acide acrylique et/ou méthacrylique et/ou de leurs esters avec des alcools mono- ou polyfonctionnels, d'acrylates et/ou méthacrylates, de vinylpyrrolidone, d'alcool vinylique, d'acétate de vinyle, d'esters vinyliques, de polyamines, de polyuréthannes et/ou de polyéthers, et les fractions peu hygroscopiques et/ou hydrophobes étant constituées de styrène, butadiène, isoprène et/ou acrylonitrile, et en ce que le revêtement polymère est solidarisé à la surface de l'article en caoutchouc par coagulation du latex de copolymère.

2. Article en caoutchouc selon la revendication 1, caractérisé en ce que le revêtement polymère comporte une substance à propriétés tensioactives, bactéricides, fongicides et/ou bactériostatiques et/ou est revêtu d'une telle substance.

3. Article en caoutchouc selon la revendication 2, caractérisé en ce que la substance tensioactive comporte des silicones, des amines d'acides gras à longue chaîne, des amines secondaires et/ou tertiaires, des amines quaternaires à propriétés bactéricides et/ou bactériostatiques, des siloxanes, des sels d'acides organiques et/ou des produits de condensation d'acides organiques ou d'alcools avec des alcools polyfonctionnels.

4. Article en caoutchouc selon l'une des revendications 2 ou 3, caractérisé en ce que l'on applique additionnellement des types d'amines quaternaires en tant que substances bactéricides, fongicides et/ou bactériostatiques.

5. Procédé de préparation d'un article en caoutchouc souple selon l'une des revendications 1 à 4, caractérisé en ce que l'on applique un agent filmogène ou un précurseur de ce dernier, par immersion ou pulvérisation, sur l'article en caoutchouc et qu'on le fait coaguler ensuite.

6. Procédé selon la revendication 5, caractérisé en ce que la coagulation est opérée par chauffage et/ou séchage.

7. Procédé selon la revendication 6, caractérisé en ce que l'on utilise des organosiloxanes comme composés thermosensibles pour la coagulation thermique.

8. Procédé selon l'une des revendications 5 à 7, caractérisé en ce que l'on ajoute à l'agent des coagulants chimiques.

9. Procédé selon la revendication 8, caractérisé en ce que l'on utilise des sels de cations multivalents comme coagulants chimiques.

10. Article en caoutchouc selon l'une des revendications 1 à 4, caractérisé en ce que l'article en caoutchouc est un gant.

11. Article en caoutchouc selon l'une des revendications 1 à 4, caractérisé en ce que l'article en caoutchouc est un préservatif.

12. Article en caoutchouc selon l'une des revendications 1 à 4, caractérisé en ce que l'article en caoutchouc est un cathéter.
